Europäisches Patentamt

European Patent Office

Office européen des brevets

(19)

(11) Veröffentlichungsnummer: **0 184 129**
**B1**

(12) # EUROPÄISCHE PATENTSCHRIFT

(45) Veröffentlichungstag der Patentschrift:
01.06.88

(51) Int. Cl.⁴: **C 07 C 53/126**, C 07 C 51/00 //
C08K5/09

(21) Anmeldenummer: 85115047.4

(22) Anmeldetag: **27.11.85**

(54) **Verfahren zur Herstellung von 2-basischen Blei(II)-fettsäuresalzen durch Schmelzreaktion.**

(30) Priorität: **05.12.84 DE 3444261**

(43) Veröffentlichungstag der Anmeldung:
**11.06.86 Patentblatt 86/24**

(45) Bekanntmachung des Hinweises auf die Patenterteilung:
**01.06.88 Patentblatt 88/22**

(84) Benannte Vertragsstaaten:
**AT BE CH DE FR GB IT LI NL**

(56) Entgegenhaltungen:
**DE - A - 1 768 450**
**DE - A - 2 652 328**
**GB - A - 684 155**

(73) Patentinhaber: **NEYNABER CHEMIE GmbH, Am Wedenberg Posfach 11 20, D-2854 Loxstedt (DE)**

(72) Erfinder: **Worschech, Kurt, Dr., Aite Strasse 4, D-2854 Loxstedt (DE)**
Erfinder: **Wedl, Peter, Brookstrasse 10, D-2854 Loxstedt (DE)**
Erfinder: **Fleischer, Erwin, Dwarsweg 1, D-2850 Bremerhaven-Spaden (DE)**
Erfinder: **Löffelholz, Frido, Vieländerweg 226 F, D-2850 Bremerhaven-Surheide (DE)**

## Beschreibung

Bei der Herstellung von Formkörpern aus halogenhaltigen Polymerisaten, insbesondere aus hartem Polyvinylchlorid werden in weitem Umfang Stabilisatoren auf Basis von Bleiverbindungen eingesetzt. Stabilisatorsysteme dieser Art sind nicht nur hochwirksam, sondern auch preiswert. Sie gewährleisten darüber hinaus einen effektiven Lichtschutz für die Fertigprodukte. Rezepturen mit Stabilisatoren auf Basis von Bleiverbindungen enthalten seit vielen Jahren üblicherweise sogenannte Primärstabilisatoren, wegen ihrer Deckkraft auch Pigmentstabilisatoren genannt. Es handelt sich hier in der Regel um basische Bleisulfate.

Als weitere Stabilisatoren kommen organische Bleisalze, insbesondere Blei-Fettsäureseifen in Betracht. Wichtig sind insbesondere die technischen Bleistearate, die entweder als 2-basisches Bleistearat $2 PbO \cdot Pb(Fettsäurerest)_2$ — 51-gewichtsprozentiges Bleistearat — oder als neutrales Bleistearat $Pb(Fettsäurerest)_2$ — 28-gewichtsprozentiges Bleistearat — zum Einsatz kommen. Häufig werden auch Kombinationen dieser beiden Bleisalze eingesetzt.

Komplettiert wird das Stabilisatorsystem zumeist durch Calciumseifen, insbesondere technisches Calciumstearat. Diese Metallseifen kann man auch dem Gleitmittelsystem zurechnen, das gewöhnlich Paraffine, gegebenenfalls freie Fettsäuren, andere Kohlenwasserstoffwachse, wie Polyethylenderivate und in einigen Fällen auch Fettsäureester enthält.

Der wirtschaftliche Zwang, nach immer preiswerteren Rezepturen zu suchen, führte in letzter Zeit dazu, Pigmentstabilisatoren vom Typ des basischen Bleisulfats mehr und mehr zu eliminieren. Auch die Weiterentwicklung auf der Seite der Kunststoffverarbeitungsmaschinen hat dazu geführt, dass mit wesentlich schwächer stabilisierten Formmassen auf Basis von halogenhaltigen Polymerisaten heute problemlos extrudiert werden kann. Als Primärstabilisatoren rücken daher die basischen Bleiseifen $2 PbO \cdot Pb(Fettsäurerest)_2$ immer stärker in den Mittelpunkt des Interesses, wobei als rheologisch wirksame Rezepturbestandteile und Co-Stabilisatoren neutrale Bleiseifen und Calciumseifen oder auch nur die neutralen Bleiseifen eingesetzt werden. An den weiterhin verwendeten Komponenten der Stabilisator-/Gleitmittelsysteme für halogenhaltige Polymere hat sich demgegenüber wenig geändert.

Stoffgemische der hier betroffenen Art bieten den Anreiz, das gesamte Stabilisierungs- und Rheologiesystem als sogenannten Schmelzcompound herzustellen. Entsprechende Vorschläge sind beispielsweise in der DE-PS 1 544 697 und der DE-PS 1 569 190 enthalten. Diese Druckschriften beschreiben nichtstäubende Stabilisator/Gleitmittelkombinationen für Vinylchloridpolymerisate, die beispielsweise aus einem in der Schmelze vereinigten Gemisch von Gleitmitteln, Metallseifen einer langkettigen aliphatischen Carbonsäure und basischen Bleisalzen anorganischer oder organischer Säuren bestehen. Nach bisherigem Wissen ist es dabei allerdings schwierig, wenn nicht unmöglich, 2-basische Bleiseifen und insbesondere definierte Mengen an 2-basischen Bleiseifen neben neutralen Bleiseifen im Schmelzverfahren herzustellen. Dementsprechend wird in diesen Druckschriften vorgeschlagen, getrennt hergestellte 2-basische Bleiseifen in die Schmelze aus Gleitmitteln und/oder neutralen Bleiseifen einzutragen.

Die Erfindung geht von der Aufgabe aus, ein einfaches Verfahren zu entwickeln, mit dem die in-situ-Herstellung von 2-basischen Blei-Fettsäuresalzen im Schmelzverfahren ermöglicht wird. Insbesondere will die Erfindung ein Schmelzverfahren zugänglich machen, bei dem vorgegebene Mengenverhältnisse von neutralen Bleiseifen und 2-basischen Bleiseifen der angegebenen Art im Schmelzverfahren zugänglich werden. Mit dem neuen Verfahren will die Erfindung einen vereinfachten Weg zur Herstellung von Stabilisatorsystemen für halogenhaltige Polymere, insbesondere für Polyvinylchlorid, schaffen, der die Vereinigung der Bestandteile mit der Herstellung wesentlicher Komponenten in der Schmelze verbindet.

Die technische Lösung der gestellten Aufgabe geht von der überraschenden Feststellung aus, dass sich basische Bleiseifen in der Schmelze aus neutralen Bleiseifen herstellen lassen, wenn in der Schmelze bestimmte Mischungskomponenten vorhanden sind.

Gegenstand der Erfindung ist dementsprechend ein Verfahren zur Herstellung von Blei(II)-fettsäuresalzen durch Umsetzung von Blei(II)-oxid mit einer Schmelze der bleisalzbildenden Fettsäuren, vor allem im Rahmen der Herstellung von Stabilisierungsgemischen für halogenhaltige Polymerisate, insbesondere für Polyvinylchlorid, das dadurch gekennzeichnet ist, dass man bei der Herstellung von 2-basischen Bleisalzen der Formel $2 PbO \cdot Pb(Fettsäurerest)_2$ mit einer Schmelze arbeitet, die mindestens eine mit halogenhaltigen Polymerisaten verträgliche Magnesium-, Calcium- und/oder Aluminiumverbindung enthält.

Im erfindungsgemässen Verfahren erfolgt die Bildung des 2-basischen Bleisalzes in einer Schmelze des neutralen Bleifettsäuresalzes oder in Schmelzen, die das neutrale Bleifettsäuresalz enthalten. In diese Schmelzen wird feinteiliges Blei(II)-oxid eingetragen. In Gegenwart der genannten Magnesium-, Calcium- und/oder Aluminiumverbindungen erfolgt eine rasche Umsetzung des Bleioxids, die sich durch das Verschwinden der gelben Oxidfarbe deutlich zu erkennen gibt. In Abwesenheit der erfindungsgemäss einzusetzenden Magnesium-, Calcium- und Aluminiumverbindungen tritt keine Reaktion mit dem eingetragenen Bleioxid ein, letzteres verbleibt vielmehr in Gestalt gelber Partikel — teils in suspendierter Form, teils als Bodenkörper — in der Schmelze.

Zur Katalysierung der Bildung des 2-basischen Bleisalzes in der Schmelzreaktion werden insbesondere die mit halogenhaltigen Polymerisaten verträglichen Verbindungen Magnesiumhydroxid, Magnesiumoxid, Magnesiumcarbonat, Calciumhydroxid, Calciumoxid, Calciumcarbonat und Aluminiumhydroxid, vorzugsweise in der jeweiligen wasserfreien Form, eingesetzt.

In einer besonders wichtigen Ausführungsform der Erfindung enthalten die Schmelzen, in denen die 2-basischen Blei(II)-fettsäuresalze hergestellt werden, Magnesium-, Calcium- und/oder Aluminiumseifen, denen wegen ihrer Stabilisator- bzw. Gleitmitteleigenschaften in Stabilisatorgemischen für halogenhaltige Polymerisate besondere Bedeutung zukommt. Bei den hier in Betracht kommenden Seifen handelt es sich um die Magnesium-, Calcium- und Aluminiumsalze von Fettsäuren mit 12 bis 24, vorzugsweise 16 bis 18 Kohlenstoffatomen. Die Kohlenstoffkette dieser Fettsäuren kann geradkettig oder verzweigt sein. In der Regel besteht die Fettsäurekomponente dieser Seifen aus den in den natürlichen Fetten und Ölen vorkommenden Fettsäuren wie Laurin-, Myristin-, Palmitin- und Stearinsäure. Bei den genannten Seifen kann es sich um Magnesium-, und Aluminiumsalze einzelner Fettsäuren handeln. Die Fettsäurekomponente dieser Seifen kann aber auch aus einem Gemisch verschiedener Fettsäuren des genannten Kohlenstoffzahlbereichs bestehen, wobei Gemischen aus Palmitinsäure und Stearinsäure, d.h. aus Fettsäuren mit 16 und 18 Kohlenstoffatomen besondere Bedeutung zukommen kann. Die genannten Magnesium-, Calcium- und Aluminiumseifen können als solche in die Schmelze für die Herstellung der 2-basischen Bleisalze eingebracht werden. Normalerweise wird es vorgezogen, diese Seifen in der für die Herstellung der 2-basischen Bleisalze verwendeten Schmelze selbst herzustellen. In diesem Fall besteht die Fettsäurekomponente der Magnesium-, Calcium- und/oder Aluminiumseifen aus der bleisalzbildenden Fettsäure, und die Seifen werden in der Schmelze durch Auflösen einer entsprechenden Menge Magnesiumhydroxid, Magnesiumoxid, Calciumhydroxid, Calciumoxid oder Aluminiumhydroxid erzeugt, bevor das für die Bildung des 2-basischen Bleisalzes aus dem neutralen Bleisalz erforderliche Bleioxid zugegeben wird.

An dieser Stelle sei festgestellt, dass die Oxide und Hydroxide des Magnesiums und Calciums und Aluminiumhydroxid offensichtlich auch dann im Sinne der Erfindung die Bildung des 2-basischen Bleisalzes katalysieren, wenn sie nicht in die entsprechenden Seifen umgewandelt werden. Zumindest tritt diese Wirkung auch dann ein, wenn die genannten Verbindungen erst in die Schmelze eingebracht werden, wenn die gesamte vorhandene Fettsäure mit Bleioxid zur neutralen Bleiseife abreagiert hat.

Die erfindungsgemäss in der Schmelze vorhandenen Magnesium-, Calcium- und/oder Aluminiumverbindungen werden zur Auslösung und Förderung der bisher nicht zugänglichen Schmelzreaktion von Blei(II)-oxid mit der neutralen Bleiseife zur 2-basischen Bleiseife nur in sehr geringen Mengen benötigt. Dabei können bereits katalytische Mengen ausreichen. Bevorzugt wird mit Schmelzen gearbeitet, die wenigstens 5 Gewichtsprozent, bezogen auf das gesamte Bleisalz im Endprodukt, Magnesium-, Calcium-, und/oder Aluminiumverbindung enthalten. Die Obergrenze des Gehaltes der Schmelzen an den genannten Verbindungen ist für das erfindungsgemässe Verfahren nicht kritisch. Bei den Oxiden und Hydroxiden des Magnesiums, Calciums und Aluminiums, die keine besonderen Wirkungen gegenüber halogenierten Polymerisaten aufweisen, arbeitet man vorzugsweise mit Mengen von 5 bis 10 Gewichtsprozent, bezogen auf das gesamte Bleisalz im Endprodukt. Bei den Magnesium-, Calcium- und Aluminiumseifen handelt es sich um Verbindungen, die gegebenenfalls im System als Stabilisator- und/oder Gleitmittelkomponenten erwünscht sind. In diesem Zusammenhang können die genannten Seifen im Endprodukt bis zu 25 Gewichtsprozent, bezogen auf das gesamte dort vorhandene Bleisalz, ausmachen. Hier können diese Seifen bei der Bildung der 2-basischen Bleiseifen durch Schmelzreaktion in der gesamten erforderlichen Menge oder in Teilmengen in der Schmelze vorhanden sein.

Im Rahmen des erfindungsgemässen Verfahrens lassen sich Gemische aus neutralen und 2-basischen Bleisalzen herstellen, in denen die 2-basischen Bleisalze bis zu 50 Gewichtsprozent der vorhandenen Bleisalze ausmachen. Die Fettsäurekomponente der Bleisalze kann aus geradkettigen und verzweigten Fettsäuren mit 12 bis 24 Kohlenstoffatomen bestehen, die vorzugsweise gesättigt sind. In der Regel besteht die Fettsäurekomponente der Bleisalze aus Fettsäuren, die in den natürlichen Fetten und Ölen vorkommen, beispielsweise aus Laurin-, Myristin-, Palmitin- und Stearinsäure. Diese Säuren können als chemische Individuen vorhanden sein. In der Regel besteht die Fettsäurekomponente aus einem Gemisch verschiedener Fettsäuren des genannten Kohlenstoffzahlbereichs. Dabei kommt Gemischen von Fettsäuren mit 16 und 18 Kohlenstoffatomen, also Palmitin- und Stearinsäure, besondere Bedeutung zu, wobei vor allem Gemische von besonderem Interesse sind, in denen die beiden Säuren zu ungefähr gleichen Gewichtsteilen vorhanden sind.

Die Herstellung von definierten Gemischen aus neutralen und 2-basischen Bleifettsäuresalzen erfolgt im einfachsten Fall durch Zugabe von Blei(II)-oxid zu Schmelzen von neutralen Blei(II)-fettsäuresalzen, die mindestens eine der genannten Magnesiums-, Calcium- und/oder Aluminiumverbindungen enthalten. In diesen Schmelzen können neben den neutralen Blei(II)-fettsäuresalzen bereits in Stabilisator-/Gleitmittelkombinationen erwünschte schmelzbare Komponenten vorhanden sein, die sich ihrerseits gegenüber dem zugesetzten Bleioxid inert verhalten, beispielsweise die für ihre Gleitmitteleigenschaften bekannten Paraffine, neutralen Fettsäureester von Mono- und Polyalkoholen, Polycarbonsäurefettalkoholester und Komplexester aus Polyalkoholen, Dicarbonsäuren und Fettsäuren oder aus Polyalkoholen, Polycarbonsäuren und Fettalkoholen.

In einer bevorzugten Ausführungsform des erfindungsgemässen Verfahrens wird zunächst das neutrale Bleisalz durch die Reaktion von Blei(II)-oxid mit der Fettsäure in der Schmelze gebildet. Dieser Schmelze wird dann die vorgesehene Magnesium-, Calcium- und/oder Aluminiumverbindung zugesetzt. Sind Magnesium-, Calcium- oder Aluminiumseifen als Zusatz zur Schmelze vorgesehen, so werden diese Seifen in der oben erwähnten Weise in der Schmelze erzeugt. Anschliessend wird in die Schmelze weiteres Blei(II)-oxid in solchen Mengen eingetragen, dass nach Abschluss der Reaktion mit dem in der zweiten Stufe zugesetzten Bleioxid die Mischung mit dem im voraus festgelegten Mengenverhältnis von neutralem zu 2-basischem Bleisalz vorliegt. Allgemein gilt, dass in bevorzugten Ausführungsformen der Erfindung im voraus definierte Gewichtsverhältnisse von neutralen Bleiseifen zu 2-basischen Bleiseifen im Bereich von 1 : 0,05 bis 1 : 1 hergestellt werden können. Bevorzugt können die Gewichtsverhältnisse im Bereich von 1 : 0,5 bis 1 : 1 sein.

Die erfindungsgemässe Schmelzreaktion kann gewünschtenfalls in Gegenwart weiterer, in der Schmelze gelöster und/oder suspendierter inerter Komponenten erfolgen, wie sie insbesondere in Stabilisator-/Gleitmittelsystemen für halogenhaltige Polymere, insbesondere für Polyvinylchlorid, üblich sind. Vor allem ist es im Rahmen der Erfindung auch möglich, den als Reaktionsprodukten anfallenden Schmelzsystemen die bei der beabsichtigten Verwendung erwünschten Komponenten beizumischen. Auf diese Weise werden nicht staubende Stabilisator-/Gleitmittelkombinationen erhalten, die in an sich bekannter Weise in Granulate oder beliebige andere feinteilige Formen überführt werden können.

Das erfindungsgemässe Verfahren hat den Vorteil, dass die Schmelzseifen umweltfreundlich hergestellt werden können, und dass die Herstellung von Stabilisatorsystemen für halogenhaltige Polymerisate, insbesondere für Polyvinylchlorid, in praktisch einem Verfahrensschritt und auf der Basis einer Bleiverbindung — nämlich Blei(II)-oxid — möglich wird. Die im Schmelzverfahren hergestellten Gemische aus neutralen und 2-basischen Bleiseifen verhalten sich anwendungstechnisch ähnlich wie entsprechende Gemische aus gefällten Seifen. Vor allem lassen sich erfindungsgemäss hergestellte Schmelzseifengranulate problemlos mit halogenhaltigen Polymerisaten in üblicher Weise, zum Beispiel in Fluidmischern, zu Trockengemischen aufbereiten. Solche Trockengemische (dry blends) können dann in bekannter Weise der formgebenden Verarbeitung zugeführt werden, beispielsweise der Herstellung von PVC-Rohren auf Doppelschneckenextrudern. Die Reststabilität des verformten Materials reicht aus, um auch die Wiederverarbeitung von Anfahrmaterial zu gewährleisten.

### Beispiele

*Beispiel 1*

In einem offenen Glasgefäss mit Rührer und Thermometer wurden 102,0 g (0,38 Mol) einer technischen $C_{16}/C_{18}$-Fettsäure (MG 270) und 27,0 g Paraffin (Smp. 75-80°C) aufgeschmolzen und auf 150°C erhitzt. Unter Rühren wurden dann im Verlauf von 2 Minuten 38,3 g (0,17 Mol) Blei(II)-oxid (Bleiglätte) in die Schmelze eingetragen, wobei die Reaktionstemperatur auf 150 - 160°C gehalten wurde. Es wurde 2 Minuten lang nachgerührt, wobei eine blanke gelbliche Schmelze entstand. Anschliessend wurden der Schmelze 1,3 g (0,02 Mol) Calciumhydroxid und 5 g Bisphenol A zugegeben. Im Verlauf von 8 Minuten wurden dann 30 g (0,13 Mol) Bleiglätte in die weiterhin auf 150 - 160°C gehaltene Schmelze eingetragen. Die Schmelze wurde dann 30 Minuten lang bei 150 bis 160°C weitergerührt. Danach hatte sich das zugesetzte Bleioxid aufgelöst; die Reaktionsmischung lag als gelblich-graustichige getrübte Schmelze ohne Bodenkörper vor, die beim Abkühlen zu einem beigefarbenen hartwachsartigen Festkörper erstarrte (Smp. 102°C).

*Beispiel 2*

Analog Beispiel 1 wurden 56,6 g (0,21 Mol) einer technischen $C_{16}/C_{18}$-Fettsäure (MG 270) und 49,5 g Hartparaffin (Smp. 100°C) durch Erhitzen auf 150°C zu einer blanken gelbstichigen Flüssigkeit aufgeschmolzen. 150 - 155°C wurden dann im Verlauf von 2 Minuten 23,4 g (0,105 Mol) Bleiglätte unter Rühren in die Schmelze eingetragen. Es wurde 2 Minuten lang weitergerührt, wobei eine blanke, gelbliche Schmelze entstand. Anschliessend wurden zunächst 4,5 g Calciumcarbonat und dann 18,0 g (0,08 Mol) Bleiglätte in die Schmelze eingetragen. Das Gemisch wurde 30 Minuten lang bei 150 - 160°C weitergerührt. Danach hatte sich das zugesetzte Bleioxid aufgelöst; das Reaktionsgemisch stellte eine gelblichbraunstichige getrübte Schmelze ohne Bodenkörper dar, das beim Abkühlen zu einem schwach karamellfarbenen wachsartigen Festkörper erstarrte (Smp. 100°C).

*Beispiel 3*

Analog Beispiel 1 wurden 92,8 g (0,34 Mol) einer technischen $C_{16}/C_{18}$-Fettsäure (MG 270) und 37,0 g Paraffin (Smp. 75 - 80°C) auf 150°C erhitzt. In die Schmelze wurden im Verlauf von 3 Minuten 38,0 g (0,17 Mol) Bleiglätte eingetragen. Es wurde 5 Minuten lang weitergerührt, wobei eine blanke, gelbliche Schmelze entstand. Anschliessend wurden 10 g Magnesiumstearat und 5 g Bisphenol A in die Schmelze eingerührt, bevor 30,0 g (0,13 Mol) Bleiglätte im Verlauf von 3 Minuten zugegeben wurden. Das Gemisch wurde 30 Minuten lang bei 150 - 160°C weitergerührt. Danach war die gelbe Farbe des zugesetzten Blei(II)-oxids verschwunden. Das Reaktionsgemisch lag als gelblich-braunstichige Schmelze ohne Bodenkörper vor, die beim Abkühlen zu einem gelblich-braunstichigen, wachsartigen Festkörper erstarrte.

*Beispiel 4*

Analog Beispiel 1 wurden 92,8 g (0,34 Mol) einer technischen $C_{16}/C_{18}$-Fettsäure (MG 270) und 27 g Paraffin (Smp. 75 - 80°C) geschmolzen und auf 150°C erhitzt. In die Schmelze wurden 38,0 g (0,17 Mol) Bleiglätte im Verlauf von 3 Minuten eingerührt und 5 Minuten lang weitergerührt. Der entstandenen blanken, gelblichen Schmelze wurden nacheinander 16 g Aluminiumstearat und 5 g Bisphenol A zugegeben. Danach wurden im Verlauf von 3 Minuten 30,0 g (0,13 Mol) Bleiglätte zugegeben und das Gemisch 60 Minuten lang bei 150 - 160°C weitergerührt. Danach hatte sich das zugesetzte Blei(II)-oxid aufgelöst. Das Reaktionsgemisch lag als bräunlich-gelbe Schmelze vor, die beim Abkühlen zu einem braunstichigen wachsartigen Festkörper erstarrte.

### Patentansprüche

1. Verfahren zur Herstellung von Blei(II)-fettsäuresalzen durch Umsetzung von Blei(II)-oxid mit einer

Schmelze der Bleisalz bildenden Fettsäuren, vor allem im Rahmen der Herstellung von Stabilisatorgemischen für halogenhaltige Polymerisate, insbesondere für Polyvinylchlorid, dadurch gekennzeichnet, dass 2-basische Bleisalze der Formel 2 PbO · Pb(Fettsäurerest)$_2$ in einer Schmelze der neutralen Blei-fettsäuresalze die mindestens eine mit halogenhaltigen Polymerisaten verträgliche Magnesium-, Calcium- und/oder Aluminiumverbindung enthält, unter Zusatz von Blei(II)-oxid hergestellt werden.

2. Verfahren nach Anspruch 1, dadurch gekennzeichnet, dass man mit einer Schmelze arbeitet, die wenigstens katalytische Mengen Magnesium-, Calcium- und/oder Aluminiumverbindungen enthält.

3. Verfahren nach den Ansprüchen 1 und 2, dadurch gekennzeichnet, dass man mit einer Schmelze arbeitet, die wenigstens 5 Gewichtsprozent, bezogen auf das gesamte Bleisalz im Endprodukt, Magnesium-, Calcium- und/oder Aluminiumverbindungen enthält.

4. Verfahren nach den Ansprüchen 1 bis 3, dadurch gekennzeichnet, dass man mit einer Schmelze arbeitet, die Magnesiumhydroxid, Magnesiumoxid, Magnesiumcarbonat, Calciumhydroxid, Calciumoxid, Calciumcarbonat und/oder Aluminiumhydroxid enthält.

5. Verfahren nach den Ansprüchen 1 bis 3, dadurch gekennzeichnet, dass man mit einer Schmelze arbeitet, die Magnesium-, Calcium- und/oder Aluminiumseifen enthält.

6. Verfahren nach Anspruch 5, dadurch gekennzeichnet, dass man mit einer Schmelze arbeitet, die Magnesium-, Calcium- und/oder Aluminiumseifen von Fettsäuren mit 12 bis 24, vorzugsweise 16 bis 18 Kohlenstoffatomen enthält.

7. Verfahren nach Ansprüchen 1 bis 6, dadurch gekennzeichnet, dass Bleisalze von Gemischen von Fettsäuren mit 16 und 18 Kohlenstoffatomen hergestellt werden.

8. Verfahren nach den Ansprüchen 1 bis 7, dadurch gekennzeichnet, dass zur Herstellung von im voraus definierten Gemischen von neutralen und 2-basischen Fettsäuresalzen zunächst neutrales Bleisalz durch Reaktion von Blei(II)-oxid mit Fettsäure gebildet wird, die vorgesehene Magnesium-, Calcium- und/oder Aluminiumverbindung der Schmelze zugesetzt oder in der Schmelze erzeugt wird, weiteres Blei(II)-oxid in die Schmelze eingetragen und die Reaktion bis zur Umwandlung des Blei(II)-oxids in basisches Bleisalz fortgeführt wird.

9. Verfahren nach den Ansprüchen 1 bis 8, dadurch gekennzeichnet, dass die im voraus definierten Gewichtsverhältnisse von neutralen Bleisalzen zu 2-basischen Bleisalzen im Bereich von 1 : 0,05 bis 1 : 1, vorzugsweise im Bereich von 1 : 0,5 bis 1 : 1 liegen.

10. Verfahren nach den Ansprüchen 1 bis 9, dadurch gekennzeichnet, dass man die Schmelzreaktion in Gegenwart weiterer in der Schmelze gelöster und/oder suspendierter inerter Komponenten von Stabilisator/Gleitmittelsystemen für halogenhaltige Polymere, insbesondere für Polyvinylchlorid durchführt.

## Claims

1. A process for the production of lead(II) fatty acid salts by reaction of lead(II) oxide with a melt of the lead-salt-forming fatty acids, above all in the production of stabilizer mixtures for halogen-containing polymers, more especially for polyvinyl chloride, characterized in that dibasic lead salts corresponding to the formula 2 PbO · Pb(fatty acid residue)$_2$ are produced in a melt of the neutral lead fatty acid salts, which contains at least one magnesium, calcium and/or aluminium compound compatible with halogen-containing polymers, with addition of lead(II) oxide.

2. A process as claimed in claim 1, characterized in that the melt used contains at least catalytic quantities of magnesium, calcium and/or aluminium compounds.

3. A process as claimed in claims 1 and 2, characterized in that the melt used contains at least 5% by weight, based on the entire lead salt in the end product, of magnesium, calcium and/or aluminium compounds.

4. A process as claimed in claims 1 to 3, characterized in that the melt used contains magnesium hydroxide, magnesium oxide, magnesium carbonate, calcium hydroxide, calcium oxide, calcium carbonate and/or aluminium hydroxide.

5. A process as claimed in claims 1 to 3, characterized in that the melt used contains magnesium, calcium and/or aluminium soaps.

6. A process as claimed in claims 5, characterized in that the melt used contains magnesium, calcium and/or aluminium soaps of $C_{12}$-$C_{24}$ and preferably $C_{16}$-$C_{18}$ fatty acids.

7. A process as claimed in claims 1 to 6, characterized in that lead salts of mixture of $C_{16}$ and $C_{18}$ fatty acids are produced.

8. A process as claimed in claims 1 to 7, characterized in that, to produce pre-defined mixtures of neutral and dibasic fatty acid salts, neutral lead salt is first formed by reaction of lead(II) oxide with fatty acid, the magnesium, calcium and/or aluminium compound envisaged is added to the melt or is produced in the melt, more lead(II) oxide is introduced into the melt and the reaction is continued until the lead(II) oxide has been converted into basic lead salt.

9. A process as claimed in claims 1 to 8, characterized in that the pre-defined ratios by weight of neutral lead salts to dibasic lead salts are from 1 : 0.05 to 1 : 1 and preferably from 1 : 0.5 to 1 : 1.

10. A process as claimed in claims 1 to 9, characterized in that the melt reaction is carried out in the presence of other inert components of stabilizer/lubricant systems for halogen-containing polymers, more especially for polyvinyl chloride, which are dissolved or suspended in the melt.

## Revendications

1. Procédé pour la préparation de sels d'acides gras et de plomb (II) par réaction d'oxyde de plomb (II) avec une masse fondue à base des acides gras formant des sels de plomb, notamment dans le cadre de la préparation de mélanges de stabilisants pour polymères halogénés, en particulier, pour polychlorure de vinyle, caractérisé en ce que l'on prépare des sels dibasique de plomb, de formule 2 PbO · Pb (radical d'acide gras)$_2$ dans une masse fondue à base des sels neutres d'acides gras et de plomb, qui contient au moins un composé à base de magnésium, calcium et/ou aluminium, compatible avec des polymères halogénés, avec addition d'oxyde de plomb (II).

2. Procédé selon la revendication 1, caractérisé en ce que l'on opère avec une masse fondue qui contient au moins des quantités catalytiques de composés à base de magnésium, calcium et/ou aluminium.

3. Procédé selon les revendications 1 et 2, caractérisé en ce que l'on opère avec une masse fondue qui contient au moins 25% en poids, par rapport à l'ensemble des sels de plomb dans le produit final, de composés à base de magnésium, calcium et/ou aluminium.

4. Procédé selon l'une quelconque des revendications 1 à 3, caractérisé en ce que l'on opère avec une masse fondue qui contient de l'hydroxide de magnésium, de l'oxyde de magnésium, des carbonates de magnésium, de l'hydroxide de calcium, de l'oxyde de calcium, du carbonate de calcium et/ou de l'hydroxyde d'aluminium.

5. Procédé selon l'une quelconque des revendications 1 à 3, caractérisé en ce que l'on opère avec une masse fondue qui contient des savons à base de magnésium, calcium et/ou aluminium.

6. Procédé selon la revendication 5, caractérisé en ce que l'on opère avec une masse fondue qui contient des savons de magnésium, calcium et/ou aluminium et d'acides gras ayant de 12 à 24, de préférence de 16 à 18 atomes de carbone.

7. Procédé selon l'une quelconque des revendications 1 à 6, caractérisé en ce que l'on prépare des sels de plomb et de mélanges d'acides gras avant 16 et 18 atomes de carbone.

8. Procédé selon l'une quelconque des revendications 1 à 7, caractérisé en ce que, pour la préparation de mélanges préétablis de sels neutres et de sels dibasiques d'acides gras, on forme d'abord un sel neutre de plomb par réaction d'oxyde de plomb (II) avec un acide gras, on ajoute à la masse fondue ou on engendre dans la masse fondue le composé prévu à base de magnésium, calcium et/ou aluminium, on introduit à nouveau de l'oxyde de plomb (II) dans la masse fondue, et on poursuit la réaction jusqu'à la conversion de l'oxyde de plomb (II) en sel basique de plomb.

9. Procédé selon l'une quelconque des revendications 1 à 8, caractérisé en ce que les rapports pondéraux, définis au préalable, des sels neutres de plomb aux sels dibasiques de plomb se situent dans la plage de 1 : 0,05 à 1 : 1, de préférence dans la plage de 1 : 0,5 à 1 : 1.

10. Procédé selon l'une quelconque des revendications 1 à 9, caractérisé en ce que l'on effectue la réaction de fusion en présence d'autres composants inertes de systèmes de stabilisants/lubrifiants pour polymères halogénés, en particulier pour polychlorure de vinyle, dissous et/ou en suspension dans la masse fondue.